# EUROPEAN PATENT APPLICATION

(11) **EP 2 123 648 A1**
(43) Date of publication of application: **25.11.2009**
(21) Application number: 08156590.5
(22) Date of filing: 20.05.2008
(51) Int. Cl.: C07D 403/04

(54) **A process for the preparation of Telmisartan.**

(71) Applicant: Chemo Ibérica, S.A., 08028 Barcelona (ES)
(72) Inventor: Rasparini, Marcello, 27010, Cura Carpignano (PV) (IT); Tufaro, Roberto, 28060, Sozzago (Novara) (IT); Castaldi, Graziano, 28072, Briona (NO) (IT)
(74) Representative: Barlocci, Anna

(57) **Abstract**

The present invention relates to a process for the preparation of Telmisartan esters and Telmisartan and intermediates for the synthesis thereof.

## Description

### Field of the invention

The present invention relates to a process for the preparation of Telmisartan esters and Telmisartan and intermediates for the synthesis thereof.

### Background of the invention

Telmisartan and its physiologically acceptable salts have valuable pharmacological properties. Telmisartan is an angiotensin-II-antagonist, which may be used to treat hypertension and cardiac insufficiency, ischaemic peripheral circulatory disorders, myocardial ischaemia (angina), to prevent the progression of cardiac insufficiency after myocardial infarct, to treat diabetic neuropathy, glaucoma, gastrointestinal diseases and bladder diseases. Telmisartan is also suitable for treating pulmonary diseases, e. g. lung oedema and chronic bronchitis, for preventing arterial restenosis after angioplasty, for preventing thickening of blood vessel walls after vascular operations, and for preventing arteriosclerosis and diabetic angiopathy. In view of the effects of angiotensin on the release of acetyl-choline and dopamine in the brain, Telmisartan is also suitable for alleviating central nervous system disorders, e. g. depression, Alzheimer's disease, Parkinson syndrome, bulimia and disorders of cognitive function.

Telmisartan is a compound of formula (Ib) 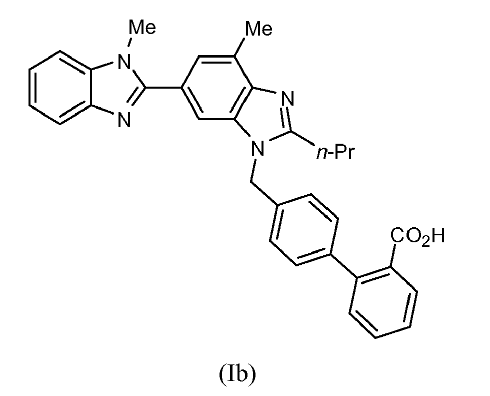 chemically known as 4'-[(1,7'-dimethyl-2'-propyl[2,5'-bis-1H-benzimidazol]-3'-yl)methyl][1,1'-biphenyl]-2-carboxylic acid, which is disclosed in EP 502 314 B1 and marketed under the trade name Micardis®.

Several methods have been used to prepare Telmisartan. Most of them are based on the alkylation of the compound of formula (VII) with biphenylcarboxylic derivatives (esters or nitriles) bearing a halomethyl group, that are in turn prepared by multistep processes.

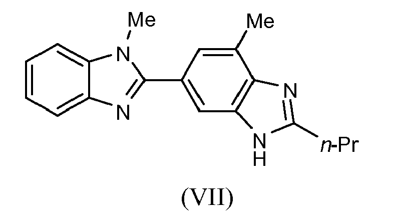

The ester or nitrile groups so obtained need then a further hydrolysis step to obtain the free acid (Ib). For instance, the patent EP 502 314 B1 describes the alkylation of the compound of formula (VII) with t-butyl 4'-(bromomethyl)biphenyl-2-carboxylate and following hydrolysis.

The patent application WO 2005108375 relates to a method for the production of Telmisartan by reacting the compound of formula (VII) with methyl-4-(bromomethyl) biphenyl-2-carboxylate and subsequently hydrolysis.

The patent application WO 2004087676 relates to a method for the production of Telmisartan by reacting the compound of formula (VII) with 4-bromomethyl-2'-cyanobiphenyl and subsequently hydrolysis of the nitrile to the free acid (Ib).

A different approach is described in the patent application EP 1719766, wherein the *N*-4-bromobenzyl derivative of the compound of formula (VII) is coupled to 2-carboxylphenyl boronic acid with a Suzuki reaction. As described in EP 1878735, 2-carboxyphenyl boronic acid requires a very laborious process to separate it, since it is extremely soluble in water, making the process unattractive for an industrial application.

An object of the present invention is to provide an efficient, economical and commercially useful process for preparing Telmisartan esters and Telmisartan and novel intermediates for the synthesis thereof.

### Summary of the invention

It has now been found a process for the preparation of Telmisartan esters and
Telmisartan in a straightforward manner exploiting novel intermediates and a metal catalyzed co-cyclization of said intermediates with acetylene.

The method of the present invention involves a process for preparing a compound of formula (I), or a pharmaceutically acceptable salt thereof, 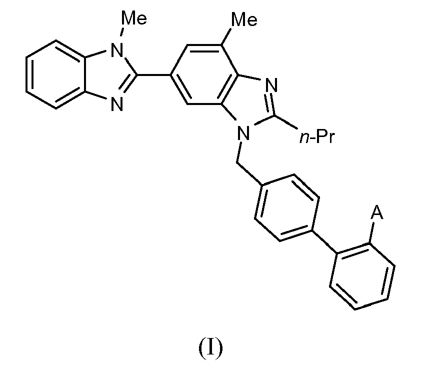 wherein A is COOH or COOR₁, where R₁ is C₁-C₆ alkyl, aryl or arylalkyl; which comprises:
the co-cyclization of a phenylpropiolic acid derivative of formula (II) or a salt thereo f, 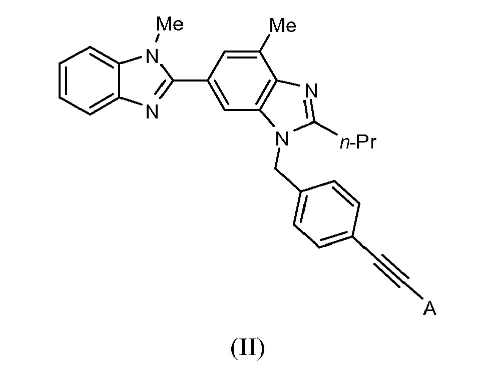
wherein A is as defined above; with acetylene (III),

H-C≡C-H (III)

in the presence of a catalyst, an optional organic ligands, in a solvent and with optional heating.

The ease of synthesis, the low cost of the reagents and the mild conditions employed make this novel process particularly suitable for industrial application.

In another aspect, the invention provides a compound of formula (II), as defined above, as well as its hydrates, solvates, and polymorphs, and the pharmaceutically acceptable salts thereof.
In another embodiment, the invention provides a compound of formula (IV), 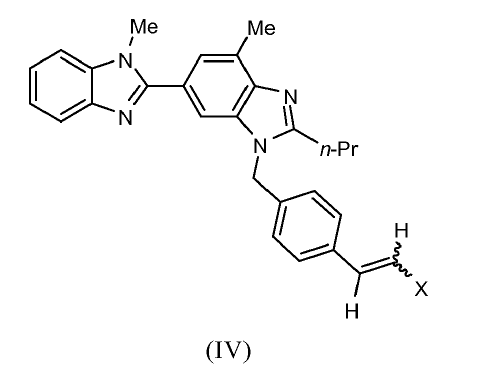 wherein X is a halogen, as well as its hydrates, solvates, and polymorphs.

In another embodiment, the invention provides the use of compounds of formula (II) and (IV), as intermediates in the preparation of Telmisartan esters or Telmisartan.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The terms "alkyl" or "C₁-C₆ alkyl" refer to a straight or branched hydrocarbon having from 1 to 6 carbon atoms, preferably 1 to 4 carbon atoms, more preferably up to 2 carbon atoms. Examples of alkyl groups include, without limitation, methyl, ethyl, *n*-propyl, isopropyl, *n*-butyl, *sec*-butyl, isobutyl, *tert*-butyl, *n*-pentyl, *n*-hexyl groups and the like. A preferred C₁-C₆ alkyl group of the present invention is methyl or ethyl.

The term "C₁-C₄ alkyl" refers to a straight or branched hydrocarbon having from 1 to 4 carbon atoms. Examples of alkyl groups include, without limitation, methyl, ethyl, *n*-propyl, isopropyl, *n*-butyl, *sec*-butyl, isobutyl, *tert*-butyl and the like. A preferred C₁-C₄ alkyl group of the present invention is methyl or ethyl.

The term "aryl" refers to an aromatic carbocyclic group having a single ring (e.g., phenyl), multiple rings (e. g., biphenyl), or multiple condensed rings in which at least one is aromatic (*e*.*g*., 1,2,3,4-tetrahydronaphthyl, naphthalen-1-yl, 2-naphthyl, anthryl, or phenanthryl). The aryl group may be optionally substituted. A preferable aryl group of the present invention is phenyl or *p*-tolyl, preferably phenyl.

The term "arylalkyl" refers to an alkyl group, preferably an alkyl group having 1 to 6 carbon atoms, substituted with an aryl group. Suitable arylalkyl groups include benzyl, tolyl or xylyl, and the like.

The term "halogen" refers to a fluorine, bromine or chlorine atom.
The term "about" encompasses the range of experimental error that may typically occur in a measurement.

The term "pharmaceutically acceptable salt(s)", as used herein, unless otherwise indicated, refers to the relatively non-toxic inorganic and organic acid-addition salts and the base-addition salts, of the compounds of the present invention. These salts may be prepared *in situ* during the final isolation and purification of the compounds.

In particular, the acid-addition salts may be prepared by separately reacting the compound with an organic or inorganic acid and isolating the salt thus formed. The resulting salts are, for example, hydrochloride, hydrobromide, hydroiodide, nitrate, sulfate, bisulfate, phosphate, dihydrogenophosphate, acid phosphate, isonicotinate, acetate, trifluoroacetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, 2-naphtalenesulfonates, camphorsulfate and pamoate [i.e., 1,1'-methylene-bis-(2-hydroxy-3-naphthoate)] salts.

The invention also relates to pharmaceutically acceptable salts with organic or inorganic bases. In particular, the basic-addition salts may be prepared by separately reacting the compound with an organic or inorganic base and isolating the salt thus formed. The resulting salts are, for example, metal salts, particularly alkali metal salts, alkaline-earth metal salts and transition metal salts (such as sodium, potassium, calcium, magnesium, aluminium), or salts obtained with bases, such as ammonia or secondary or tertiary amines (such as diethylamine, triethylamine, *tert*-butylamine, piperidine, piperazine, morpholine), or with basic amino-acids, for example lysine, or with osamines (such as meglumine), or with aminoalcohols (such as 3-aminobutanol and 2-aminoethanol).

The term "hydrate" refers to a solvate comprising a disclosed or claimed compound and a stoichiometric or non-stoichiometric amount of water.

The term "solvate" refers to a molecular complex comprising a disclosed or claimed compound and a stoichiometric or non-stoichiometric amount of one or more solvent molecules (*e*.*g*., ethanol).

Generally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible and inert solvents, which may influence the reaction rate and yield. Depending on the nature of the reactants, the one or more solvents may be polar protic solvents, polar aprotic solvents, non-polar solvents, or some combination. Suitable solvents are water; an alcohol, such as methanol, ethanol, *i*-propanol, 1- or 2- or *tert*-butanol; an ester, such as ethyl acetate or isopropyl acetate; an ether, such as tetrahydrofuran, 2-methyltetrahydrofuran or 1,4-dioxane; an aliphatic or aromatic hydrocarbon, such as hexane or heptane, toluene or xylenes; dimethylformamide, dimethylacetamide or N-methylpyrrolidone; or mixtures thereof.

The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

The process of the invention for making Telmisartan esters or Telmisartan of formula (I) is illustrated in the following reactions Scheme 1. 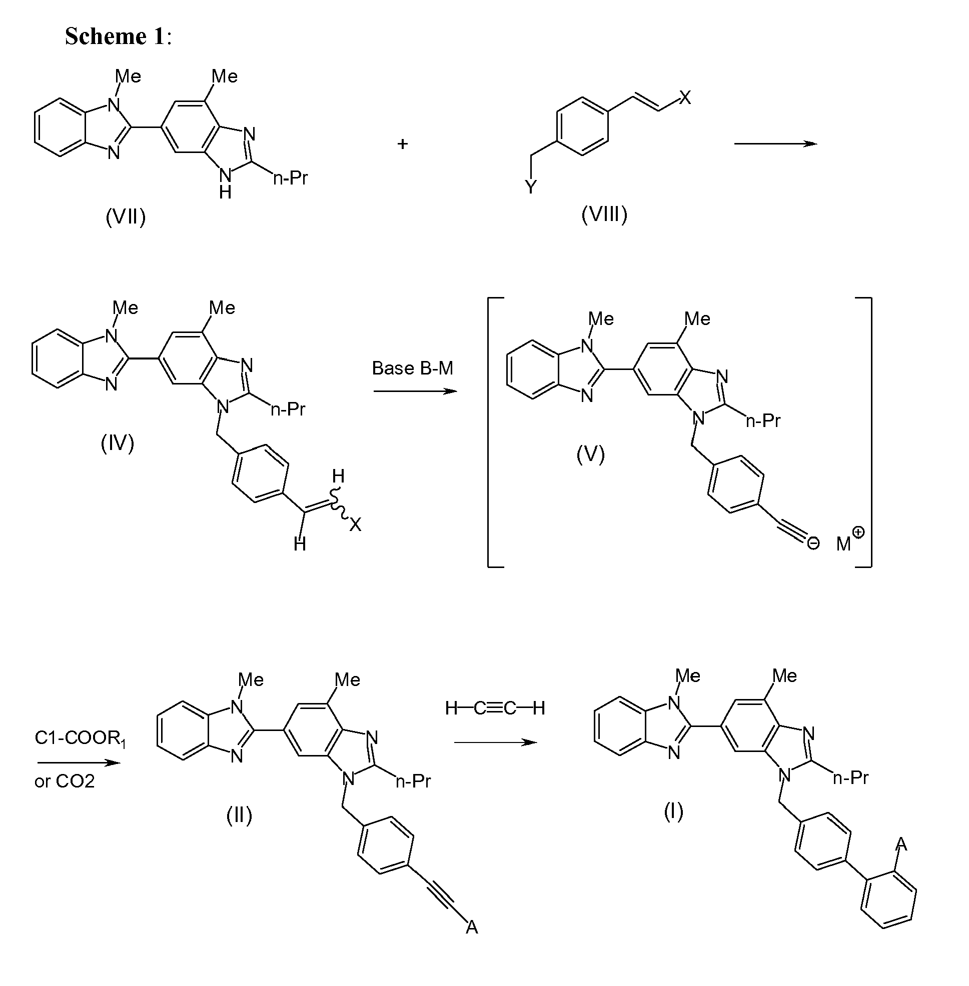 wherein:
X is a halogen;
Y is a leaving group;
B is a C₁-C₆ alkyl, aryl or R₁O-, where R₁ is C₁-C₆ alkyl, aryl or arylalkyl;
M is Li, Na, K or MgX;
R₁ is defined as above;
A is COOH or COOR₁, where R₁ is as defined above.

The process comprises the co-cyclization of a phenylpropiolic acid derivative of formula (II) or a salt thereof, with acetylene (III),

H-C≡C-H (III)

in the presence of a catalyst and optional organic ligands, in a solvent and with optional heating to produce Telmisartan esters or Telmisartan (I), wherein A is as defined above, preferably A is COOH, or a salt thereof (Scheme 1). A salt of compounds of formula (I) or (II) is for example a salt with a pharmaceutically acceptable organic or inorganic base or acid. Preferably, the sodium, potassium, magnesium or calcium salt, or the hydrochloride, hydrobromide, tosylate and camphorsulfate salt. A salt of a compound of formula (I) or (II) may be obtained employing conventional methods well known to those skilled in the art.

The co-cyclization of a compound of formula (II) with acetylene can be carried out by contacting (II) with acetylene gas, applied at pressure ranging from about atmospheric pressure to about 5 bar, preferably at atmospheric pressure.

A catalyst is typically a metal catalyst, such as Fe, Co, Ni, Pd, Rh, Ru, Ir or their salts, such as chloride, bromide, iodide, acetate, acetylacetonate (acac); or a metal complex, Ni(0) complexes, such as Ni(cod)₂ (wherein cod is 1,5-cyclooctadiene), Ni(acac)₂ or Ni(O)-phosphine complexes; Fe(0) complexes, Pd(0) complexes, such as Pd(Ph₃P)₄; Rh(I) complexes, such as RhCl(PPh₃)₃ (Wilkinson's catalyst); Ru complexes, such as PhCH=Ru(PCy₃)Cl₂ (Grubbs catalyst), where Cy is ciclohexyl; or Ir complex, such as [Ir(cod)(acac)] or [Ir(cod)Cl]₂. Preferably, a catalyst is Fe(0) complexes, Ni(acac)₂, Ni(Ph₃P)₄ or [Ir(cod)Cl]₂.

With standard methods well known to a person skilled in the art, the metal complexes can be prepared and isolated in a separate step, or can be prepared *in situ*.

A preferred method for preparing Ni(0) or Fe(0) complexes is to reduce Ni(II), Fe(II) or Fe(III) salts, such as NiZ₂, FeZ₂ or FeZ₃ salts (where Z is acac, acetate, Cl, Br or I), with a suitable reducing agent, for example Zn dust, *n*-BuLi, Et₂Zn, *i*-Bu₂AlH or di-*i*-Bu₂AlH, optionally in the presence of an organic ligand.

More preferably, Ni(0) complexes are prepared by reduction of Ni(II) salts with *n*-BuLi, in the presence of triphenylphosphine; and Fe(0) complexes are prepared by reduction of Fe(II) or Fe(III) salts with *i*-Bu₂AlH.

The catalyst is added in amounts from about 0.5% to 10 % molar equivalents, preferably from 0.5% to 5% molar equivalents.

An organic ligand is typically a phosphine, such as tricyclohexylphosphine, triphenylphosphine; or bidentate phosphines, such as 1,2-bis(diphenylphosphino)ethane (dppe), 1,3-bis(diphenylphosphino)propane (dppp), 1,4-bis(diphenylphosphino)butane (dppb). Preferably, an organic ligand is triphenylphosphine or dppe.

The reaction can be carried out in polar aprotic solvent, or non-polar solvent or some combination, preferably ethers, such as tetrahydrofuran or dioxane, more preferably tetrahydrofuran; or an aromatic hydrocarbon, such as benzene, toluene or xylenes, more preferably toluene, at a temperature ranging from about 25 °C to the reflux temperature of the reaction mixture, preferably at about 25 °C.

When the co-cyclization with acetylene (III) is carried out with a phenylpropiolic acid derivative of formula (II), wherein A is COOR₁, as defined above, Telmisartan esters of formula (I), wherein A is COOR₁, are obtained. Telmisartan esters may be converted to Telmisartan (Ib), by hydrolysis, employing a conventional method well known to those skilled in the art. Hydrolysis under acidic or under basic conditions are disclosed, for instance, respectively in US patent No. 5,591,762 and in CN patent application No. 1344712.

When the co-cyclization with acetylene (III) is carried out with a phenylpropiolic acid derivative of formula (II), wherein A is COOH, Telmisartan (Ib) is obtained.

In a preferred embodiment, the carboxylic ester of the formula (II), wherein A is COOR₁, is first converted to the acid of the formula (II), wherein A is COOH, by hydrolysis, employing a conventional method well known to those skilled in the art, for instance, with a base, such as sodium, potassium or lithium hydroxide, in a suitable solvent, for instance an alcohol, such as methanol or ethanol, and then co-cyclized with acetylene, with the method as described above, to obtain Telmisartan (Ib).

The compounds of the general formula (II) are novel compounds and are also object of the present invention.

In another embodiment, the present invention provides a compound of the general formula (II), as defined above, as well as its hydrates, solvates, and polymorphs, and the pharmaceutically acceptable salts thereof.

Preferred examples of the compounds of formula (II) are compounds wherein A is COOH, or their salts, or COOR₁, where R₁ is C₁-C₄ alkyl, aryl.

Preferred examples of the compounds of formula (II) are selected from:
3-(4-((1,7'-dimethyl-2'-propyl-1H,3'H-2,5'-bibenzo[d]imidazol-3'-yl)methyl)phenyl) propiolic acid;
sodium 3-(4-((1,7'-dimethyl-2'-propyl-1H,3'H-2,5'-bibenzo[d]imidazol-3'-yl)methyl)phenyl) propiolate;
potassium 3-(4-((1,7'-dimethyl-2'-propyl-1H,3'H-2,5'-bibenzo[d]imidazol-3'-yl)methyl)phenyl) propiolate;
magnesium 3-(4-((1,7'-dimethyl-2'-propyl-1H,3'H-2,5'-bibenzo[d]imidazol-3'-yl)methyl)phenyl) propiolate;
methyl 3-(4-((1,7'-dimethyl-2'-propyl-1H,3'H-2,5'-bibenzo[d]imidazol-3'-yl)methyl)phenyl) propiolate;
ethyl 3-(4-((1,7'-dimethyl-2'-propyl-1H,3'H-2,5'-bibenzo[d]imidazol-3'-yl)methyl)phenyl) propiolate;
isopropyl 3-(4-((1,7'-dimethyl-2'-propyl-1H,3'H-2,5'-bibenzo[d]imidazol-3'-yl)methyl)phenyl) propiolate;
butyl 3-(4-((1,7'-dimethyl-2'-propyl-1H,3'H-2,5'-bibenzo[d]imidazol-3'-yl)methyl)phenyl) propiolate;
phenyl 3-(4-((1,7'-dimethyl-2'-propyl-1H,3'H-2,5'-bibenzo[d]imidazol-3'-yl)methyl)phenyl) propiolate;
as well as its hydrates, solvates, and polymorphs, and the pharmaceutically acceptable salts thereof.

A compound of formula (II) may be obtained by reaction of a compound of formula (IV), 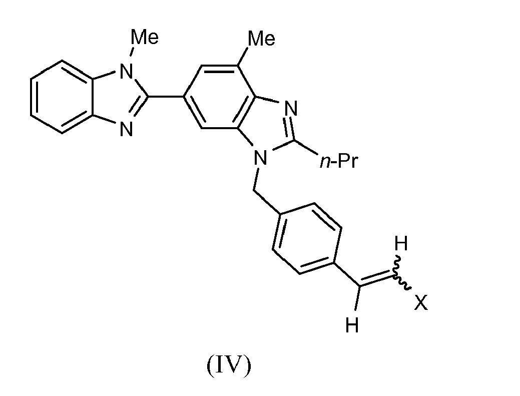 wherein X is a halogen,
with a strong base B-M,
wherein:
B is a C₁-C₆ alkyl, aryl or R₁O-, where R₁ is C₁-C₆ alkyl, aryl or arylalkyl; M is Li, Na, K or MgX;
   in a solvent, to obtain an acetylide of formula (V) 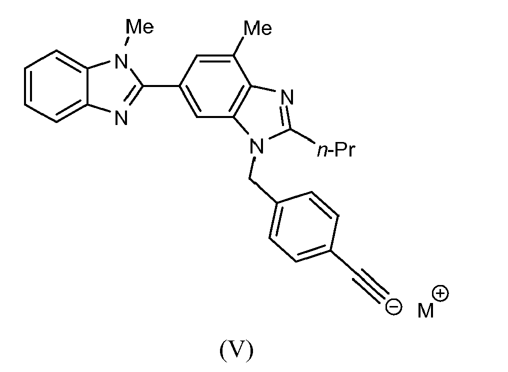
wherein M is as defined above;
followed by reaction with a chloroformate of formula (VI) 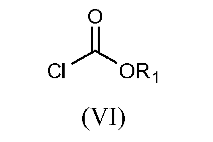 wherein R₁ is defined as above,
or, alternatively, with CO₂.

A preferred strong base B-M is an alkyl or aryl metal, such as methyllithium, butyllithium, *sec*-butlyllithium, *tert*-butyllithium, hexyllithium or phenyllithium, more preferably hexyllithium; or an alkoxyde, such as lithium, sodium or potassium methoxide, ethoxide or *tert*-butoxide, more preferably sodium or potassium *tert*-butoxide; or a Grignard reagent, such as methylmagnesium chloride or bromide, ethylmagnesium chloride or bromide, isopropylmagnesium chloride or bromide, cyclohexylmagnesium chloride or bromide or phenylmagnesium chloride or bromide, more preferably isopropylmagnesium chloride.

The strong base is added in a molar ratio to compound of formula (IV) comprised from 2.0 to 3.0, preferably from 2.0 to 2.5, in a solvent, such as an ether, for example diethyl ether, tetrahydrofuran, 2-methyltetrahydrofuran, dioxane or *tert*-butyl methyl ether, preferably tetrahydrofuran.

The reaction between a compound of formula (IV) and a base to obtain an acetylide of formula (V) is run at a temperature ranging from -78 °C to room temperature, preferably from -25 °C to room temperature.

After formation of the acetylide of formula (V), the reaction mixture of the acetylide of formula (V) is straight reacted with:
a) a chloroformate of formula (VI); or, alternatively, with:
b) CO₂, that can be added as a gas, liquid or solid; to give a compound of formula (II), wherein A is as defined above.

When a chloroformate of formula (VI) is added to the reaction mixture of the acetylide of formula (V) (Method a)), esters of a compound of formula (II) are obtained, wherein A is COOR₁. The free acid of formula (II), wherein A is COOH, can be optionally obtained by hydrolysis of the esters of a compound of formula (II), wherein A is COOR₁, and subsequently acidification.

Alternatively, when CO₂ is added to the reaction mixture of the acetylide of formula (V) (Method b)), salts of compound (II) are obtained, from which the free acid of formula (II), wherein A is COOH, can be, optionally, obtained by acidification.

The compounds of the general formula (IV) are novel compounds and are also object of the present invention.

In another embodiment, the present invention provides a compound of the general formula (IV), defined as above, as well as its hydrates, solvates, and polymorphs.

A compound of formula (IV) can be prepared by reaction of a compound a formula (VII), 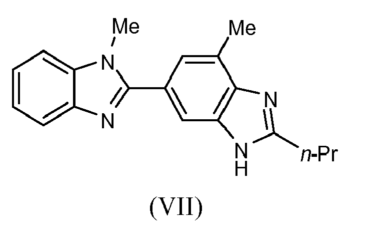 with a compound of formula (VIII), 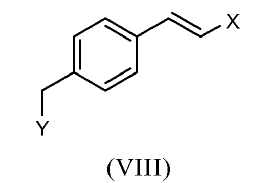 wherein X is a halogen and Y is a leaving group, in the presence of a base and in a suitable solvent.

A leaving group is typically a halogen atom, preferably bromine, or a hydroxyl group activated by esterification with an alkyl or arylsulfonyl group, such as methanesulfonyl, trifluoromethanesulfonyl, toluenesulfonyl, benzenesulfonyl.

A base can be an inorganic base, such as sodium or potassium carbonate, preferably potassium carbonate.

The reaction can be carried out in the presence of polar aprotic solvents, non-polar solvents, or some combination, such as tetrahydrofuran, acetonitrile, dimethylformamide, dimethylacetamide or N-methylpyrrolidone, preferably dimethylacetamide.

The reaction can be carried out at a temperature ranging from 0°C to 40°C, preferably at about 25°C.

The compounds useful according to the invention may be prepared, unless specifically specified, by the application or adaptation of known methods, by which are meant methods used heretofore or described in the literature, patents or patent applications, the Chemical Abstracts and on the Internet.

Compound (VII) is commercially available and compounds (VIII), where X is Cl or Br and Y is Br are known, and can be prepared as taught by Rodriguez et al. in Tetrahedron, (2003), 59(7), 1021-1032.

Compounds of formula (II), (IV) are intermediates for the preparation of pharmaceutically effective Telmisartan or Telmisartan esters.

In another embodiment, the invention provides use of compounds of formula (II), (IV), defined as above, as intermediates in the preparation of Telmisartan esters or Telmisartan.

Many of the compounds described in this disclosure, are capable of forming pharmaceutically acceptable salts. These salts include, without limitation, acid addition salts (including diacids) and base salts.

It is contemplated that the compounds of the present method can be found or isolated in the form of hydrates or solvates, in different polymorphic forms, i.e., different crystalline forms, which are considered to fall within the scope of the present invention.

The process of the invention for the preparation of the above described Telmisartan intermediates is particularly advantageous for the production on an industrial scale.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
AcOEt (ethyl acetate), dppe (1,2-bis(diphenylphosphino)ethane), DMA (N,N-dimethylacetamide), THF (tetrahydrofuran).

### Example 1 Ethyl 3-(4-((1,7'-dimethyl-2'-propyl-1H,3'H-2,5'-bibenzo[d]imidazol-3'-yl)methyl)phenyl)propiolate (IIa)

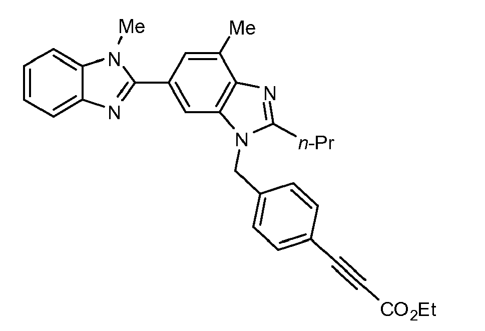

In a 100 mL two-necked round bottomed flask fitted with a thermometer, nitrogen inlet, 1,7'-dimethyl-2'-propyl-1H,3'H-2,5'-bibenzo[d]imidazole (VII) (5.0 g, 16.4 mmol), potassium carbonate (3.45 g, 25 mmol) and DMA (50 mL) were charged, followed by (*E*)-1-(bromomethyl)-4-(2-bromovinyl)benzene (VIIIa) (4.52 g, 16.4 mmol). The reaction mixture was stirred at 30 °C overnight, then diluted with toluene (100 mL) and washed with water until complete removal of the DMA. After washing with brine, the toluene extract was dried over sodium sulfate and concentrated to a residue, which was used in the following step without further purification.

The oil (theoretical 16.4 mmol) was taken up in dry THF (50 ml) and transferred to a 100 mL two-necked round bottomed flask fitted with a thermometer, nitrogen inlet and a rubber septum. The solution was cooled to -78 °C in an acetone-dry ice bath and added dropwise with n-butyllithium (21.2 mL of a 1.6M solution in hexanes, 34 mmol) keeping the temperature below -65 °C. The reaction mixture was then allowed to warm to 0 °C over 3 hours. The reaction mixture was cooled to -25 °C and ethyl chloroformate (1.7 mL, 18.0 mmol) was added dropwise, keeping the temperature below -10 °C. After complete addition, the reaction mixture was allowed to warm to room temperature overnight, then quenched with saturated ammonium chloride. THF was removed under reduced pressure and the product was extracted with AcOEt; the aqueous layer was washed with brine and dried over sodium sulfate.

By crystallization from heptane-AcOEt, the product (IIa) (4.1 g) was obtained. Overall yield: 51 %.

¹H NMR (300 MHz, CDCl₃) 1.01 (t, J=7.3 Hz, 3H, C*H*₃CH₂CH₂), 1.32 (t, J=7.0 Hz, 3H, C*H*₃CH₂O), 1.89 (sextet, J=7.3 Hz, 2H, CH₃C*H*₂CH₂), 2.76 (s, 3H, ArCH₃), 2.85 (t, J=7.3 Hz, 2H, CH₃CH₂C*H*₂) , 3.77 (s, 3H, NCH₃), 4.26 (q, J=7.0 Hz, CH₃C*H*₂O), 5.40 (s, 2H, ArCH₂N), 7.03 (d, J=8.3 Hz, 2H, ArH *metha* to C≡C), 7.25-7.40 (m, 3H, ArH), 7.42 (s, 2H, ArH), 7.50 (d, J=8.3 Hz, 2H, ArH *ortho* to C≡C), 7.76-7.77 (m, 1H, ArH).

¹³C NMR (75 MHz, CDCl₃) 14.1, 17.0, 21.9, 29.9, 31.9, 47.0, 62.3, 81.0, 85.2, 108.8, 109.6, 119.6, 122.5, 122.7, 124.0, 124.2, 126.4, 129.7, 133.7, 135.1, 138.7, 154.1, 154.6, 156.4

### Example 2 3-(4-((1,7'-Dimethyl-2'-propyl-1H,3'H-2,5'-bibenzo[d]imidazol-3'-yl)methyl)phenyl)propiolic acid (IIb)

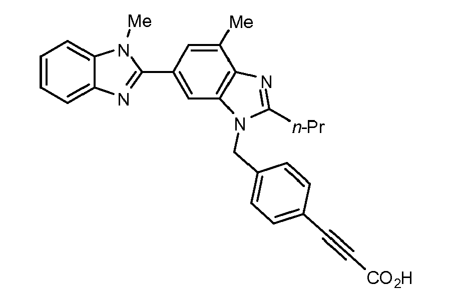

The ethyl ester (IIa), as prepared in Example 1, (2.0 g, 4.1 mmol) was dissolved in ethanol (10 mL) and water (3 mL). Sodium hydroxide (50% in water, 480 mg, 6.0 mmol) was added and the mixture stirred at 40 °C for 8 hours. Ethanol was evaporated under reduced pressure, the pH adjusted to 3 with dilute HCl and the product was extracted into AcOEt. The extract was washed with brine, dried over sodium sulfate and evaporated to dryness to yield the title product (IIb) (1.57 g), as amorphous off-white foam.
Yield: 85%.

¹H NMR (300 MHz, *d₆*-DMSO) 0.81 (t, J=7.3 Hz, 3H, C*H₃*CH₂CH₂), 1.83 (sextet, J=7.3 Hz, 2H, C*H*₃C*H₂*CH₂), 2.63 (s, 3H, ArCH₃), 2.85 (t, J=7.3 Hz, 2H, CH₃CH₂C*H₂*) , 3.80 (s, 3H, NCH₃) , 5.63 (s, 2H, ArCH₂N), 7.05 (d, J=8.3 Hz, 2H, ArH *metha* to C≡C), 7.25-7.37 (m, 3H, ArH), 7.40 (s, 2H, ArH), 7.51 (d, J=8.3 Hz, 2H, ArH *ortho* to C≡C), 7.76-7.80 (m, 1H, ArH).

### Example 3 3-(4-((1,7'-Dimethyl-2'-propyl-1H,3'H-2,5'-bibenzo [d]imidazol-3'-yl)methyl)phenyl)propiolic acid (IIb)

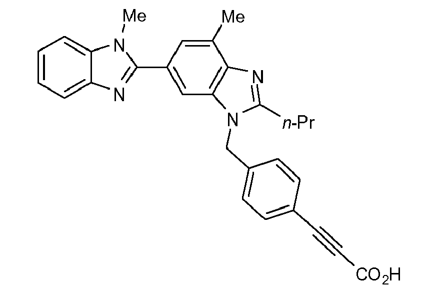

In a 100 mL two-necked round bottomed flask fitted with a thermometer, nitrogen inlet, 1,7'-dimethyl-2'-propyl-1H,3'H-2,5'-bibenzo[d]imidazole (VII) (5.0 g, 16.4 mmol), potassium carbonate (3.45 g, 25 mmol) and DMA (50 mL) were charged, followed by (*E*)-1-(bromomethyl)-4-(2-bromovinyl)benzene (VIIIa) (4.52 g, 16.4 mmol). The reaction mixture was stirred at 30 °C overnight, then diluted with toluene (100 mL) and washed with water until complete removal of the DMA. After washing with brine, the toluene extract was dried over sodium sulfate and concentrated to a residue, which was used in the following step without further purification.

The oil (theoretical 16.4 mmol) was taken up in dry THF (50 ml) and transferred to a 100 mL two-necked round bottomed flask fitted with a thermometer, nitrogen inlet and a rubber septum. The solution was cooled to -25 °C and treated portionwise with potassium *tert*-butoxide (4.05 g, 36.0 mmol), keeping the temperature below -10 °C during the additions, then the reaction mixture was warmed to room temperature and after 2 hours was poured over an excess of solid CO₂. After complete evaporation of the dry ice, the resulting mixture was diluted with AcOEt, and diluted HCl was added until pH =3 was reached. After phase separation, the aqueous layer was back-extracted with more AcOEt, the combined organic layers washed with brine, dried over sodium sulfate and concentrated to dryness to obtain the title product (IIb) (4.2 g), as a off-white foam.
Overall yield: 55%

### Example 4 Ethyl 4'-((1,7'-dimethyl-2'-propyl-1H,3'H-2,5'-bibenzo[d]imidazol-3'-yl)methyl)biphenyl-2-carboxylate (Ia)

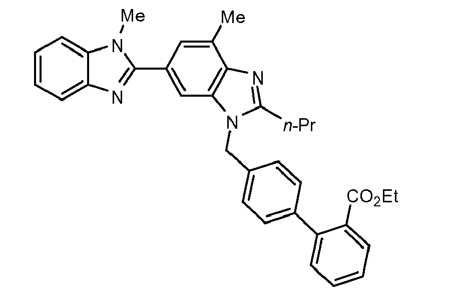

In a 150 mL two-necked round bottomed flask fitted with a stopper, nitrogen inlet and connected to a vacuum line, was charged ethyl 3-(4-((1,7'-dimethyl-2'-propyl-1H,3'H-2,5'-bibenzo[d]imidazol-3'-yl)methyl)phenyl)propiolate (IIa) (1.6 g, 3.26 mmol) and THF (70 mL). The solution was degassed with three vacuum-nitrogen purges, then [Ir(cod)Cl]₂ (13 mg, 0.02 mmol) and dppe (17 mg, 0.04 mmol) were added. The stopper was replaced with an acetylene balloon and three vacuum-acetylene cycles were performed and the mixture was stirred at 30 °C for 3 hours. THF was removed under reduced pressure and the resultant oil was purified by flash chromatography, eluting with 5% MeOH in AcOEt, obtaining the title product (Ia) (1.3 g), as colourless oil.
Yield: 73%

The same product was obtained by employing Ni(Ph₃P)₄ as catalyst in the following manner:
in a 100 mL two-necked round bottomed flask fitted with a stopper, nitrogen inlet and connected to a vacuum line Ni(acac)₂ (41.8 mg, 0.16 mmol) and Ph₃P (171.0 mg, 0.65 mmol) were dissolved in anhydrous THF (20 mL) and Dibal-H (1.0 M in THF) (0.32 mL, 0.32 mmol) was added via syringe at 0 °C. The yellow Ni(II) solution turned black. To the latter solution was added ethyl 3-(4-((1,7' dimethyl-2'-propyl-1H,3'H-2,5'-bibenzo[d]imidazol-3'
yl)methyl)phenyl)propiolate (IIa) (1.6 g, 3.26 mmol) in THF (30 mL). The nitrogen inlet was changed with an acetylene balloon and three vacuum-acetylene cycles were performed; the mixture was stirred for 4 hours at room temperature. The solvent was evaporated under reduced pressure and the resultant oil was directly purified by flash chromatography, eluting with 5% MeOH in AcOEt, obtaining the title product (Ia) (1.42 g), as colourless oil.
Yield: 79%.

The same product was obtained by employing unligated Fe(0) as catalyst in the following manner:
in a 100 mL two-necked round bottomed flask fitted with a stopper, nitrogen inlet and connected to a vacuum line FeCl₂ (20.3 mg, 0.16 mmol) was dissolved in anhydrous THF (20 mL) and Dibal-H (1.0 M in THF) (0.32 mL, 0.32 mmol) was added via syringe at 0 °C. The yellow Fe(II) solution turned black. To the latter solution was added ethyl 3-(4-((1,7'-dimethyl-2'-propyl-1H,3'H-2,5'-bibenzo[d]imidazol-3'-yl)methyl)phenyl)propiolate (IIa) (1.6 g, 3.26 mmol) in THF (30 mL). The nitrogen inlet was changed with an acetylene balloon and
three vacuum-acetylene cycles were performed; the mixture was stirred for 4 hours at room temperature. The solvent was evaporated under reduced pressure and the resultant oil was directly purified by flash chromatography, eluting with 5% MeOH in AcOEt, obtaining the title product (Ia) (0.93 g), as colourless oil.
Yield 52%.
¹H NMR (300 MHz, CDCl₃) 0.94 (t, J=7.6 Hz, 3H, C*H₃*CH₂CH₂), 1.05 (t, J=7.0 Hz, 3H, C*H₃*CH₂O), 1.87 (sextet, J=7.6 Hz, 2H, CH₃C*H₂*CH₂), 2.76 (s, 3H, ArCH₃), 2.92 (t, J=7.6 Hz, 2H, CH₃CH₂C*H₂*), 4.00 (q, J=7.0 Hz, CH₃C*H₂*O), 5.44 (s, 2H, ArCH₂N), 7.07 (d, J=8.3 Hz, 2H, ArH), 7.20-7.51 (m, 10H, ArH), 7.77-7.82 (m, 2H, ArH)
¹³C NMR (75 MHz, CDCl₃) 13.8, 14.2, 17.0, 21.9, 29.9, 31.2, 47.2, 60.9, 109.2, 109.6, 119.5, 122.6, 122.7, 123.9, 125.9, 127.5, 129.2, 129.6, 129.9, 130.7, 131.3, 134.7, 154.0, 154.6, 156.6, 168.4

### Example 5 4'-((1,7'-dimethyl-2'-propyl-1H,3'H-2,5'-bibenzo[d]imidazol-3'-yl)methyl) biphenyl-2-carboxylic acid (Ib) (Telmisartan)

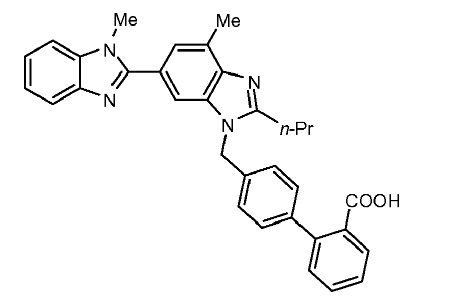

In a 50 mL two-necked round bottomed flask fitted with a stopper, nitrogen inlet and connected to a vacuum line, 3-(4-((1,7'-dimethyl-2'-propyl-1H,3'H-2,5'-bibenzo[d]imidazol-3'-yl)methyl)phenyl)propiolic acid (IIb) (1.5 g, 3.20 mmol) and THF (50 mL) were charged. The solution was degassed with three vacuum-nitrogen purges, then [Ir(cod)Cl]₂ (13 mg, 0.02 mmol) and dppe (17 mg, 0.04 mmol) were added. The stopper was replaced with an acetylene balloon and three vacuum-acetylene cycles were performed and the mixture was stirred at 30 °C for 8 hours. THF was removed under reduced pressure and the resultant oil was purified by flash chromatography eluting with 5% MeOH and 1% AcOH in AcOEt, obtaining the title product (Ib) (1.12 g), as an off-white solid.
Yield: 68%.
¹H NMR (300 MHz, *d₆*-DMSO) 0.99 (t, J=7.6 Hz, 3H, C*H₃*CH₂CH₂), 1.81 (sextet, J=7.6 Hz, 2H, CH₃C*H₂*CH₂), 2.62 (s, 3H, ArH), 2.92 (t, J=7.6 Hz, 2H, CH₃CH₂C*H₂*), 3.81 (s, 3H, NCH₃), 5.61 (s, 2H, NCH₂Ar), 7.16 (d, J=8.2 Hz, 2H, ArH), 7.21-7.34 (m, 5H, ArH), 7.42 (td, J=7.3 & 1.5 Hz, 1H, ArH), 7.47 (s, 1H, ArH), 7.53 (td, J=7.3 & 1.5 Hz, 1H, ArH), 7.55-7.58 (m, 1H, ArH), 7.65 (dd, J=6.4 & 1.5 Hz, ArH), 7.70 (dd, J=7.9 & 1.2 Hz, 2H, ArH)
¹³C NMR (75 MHz, *d₆*-DMSO) 14.4, 17.0, 21.3, 29.3, 32.2, 46.7, 109.9, 111.0, 119.2, 122.4, 122.6, 123.7, 123.8, 127.0, 127.9, 128.8, 129.3, 129.7, 130.9, 131.4, 132.8, 135.3, 136.5, 137.1, 140.7, 141,1, 142.9, 143.3, 154.6, 156.8, 170.1

## Claims

1. A process for preparing a compound of formula (I), or a pharmaceutically acceptable salt thereof, 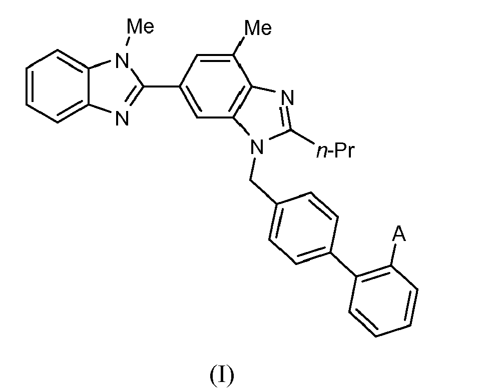 wherein A is COOH or COOR₁, where R₁ is C₁-C₆ alkyl, aryl or arylalkyl; which comprises:
the co-cyclization of a phenylpropiolic acid derivative of formula (II) or a salt thereof, 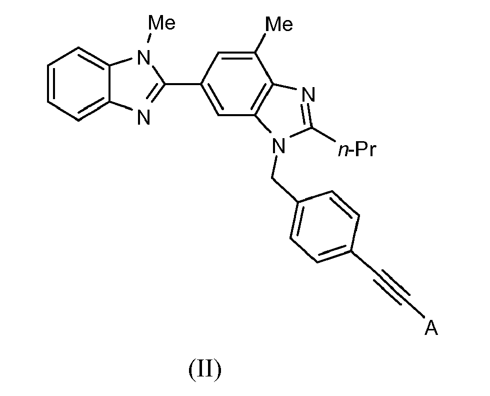
wherein A is as defined above; with acetylene (III),
H-C≡C-H (III)
in the presence of a catalyst and optional organic ligands, in a solvent and with optional heating.

2. The process according to claim 1, wherein A is COOH.

3. The process according to claim 1, wherein the catalyst is a metal, a metal salt or a metal complex.

4. The process according to claim 3, wherein the catalyst is Fe, Co, Ni, Pd, Rh, Ru, Ir or their salts, such as chloride, bromide, iodide, acetate, acetylacetonate; or Ni(0) complexes, such as Ni(cod)₂, Ni(acac)₂, Ni(0)-phosphine complexes; Fe(0) complexes, Pd(0) complexes, such as Pd(Ph₃P)₄; Rh(I) complexes, such as RhCl(PPh₃)₃; Ru complexes, such as PhCH=Ru(PCy₃)Cl₂; Ir complex, such as [Ir(cod)(acac)] or [Ir(cod)Cl]₂; preferably Fe(0) complexes, Ni(acac)₂, Ni(Ph₃P)₄ or [Ir(cod)Cl]₂.

5. The process according to claim 4, wherein Ni(0) or Fe(0) complexes are obtained by reduction of Ni(II), Fe(II) or Fe(III) salts, such as NiZ₂, FeZ₂ or FeZ₃ salts, where Z is acac, acetate, Cl, Br or I, with a reducing agent, such as Zn dust, *n*-BuLi, Et₂Zn, *i*-Bu₂AlH or di-*i*-Bu₂AlH, optionally in the presence of an organic ligand.

6. The process according to claim 5, wherein Ni(0) complexes are prepared by reduction ofNi(II) salts with *n*-BuLi, in the presence of triphenylphosphine.

7. The process according to claim 5, wherein Fe(0) complexes are prepared by reduction of Fe(II) or Fe(III) salts with *i*-Bu₂AlH.

8. The process according to claim 1, wherein the organic ligand is a phosphine, such as tricyclohexylphosphine, triphenylphosphine; or bidentate phosphines, such as dppe, dppp, dppb; preferably triphenylphosphine or dppe.

9. The process according to claim 1, wherein the solvent is a polar aprotic solvent, or non-polar solvent or some combination, preferably an ether or an aromatic hydrocarbon, more preferably tetrahydrofuran or toluene.

10. The process according to claim 1, which further comprises converting a carboxylic ester of the formula (II) or (I), wherein A is COOR₁, to the acid of the formula (II) or (I), wherein A is COOH, by hydrolysis.

11. The process according to claim 1, wherein said compound of formula (II) is prepared by reaction of a compound of formula (IV), 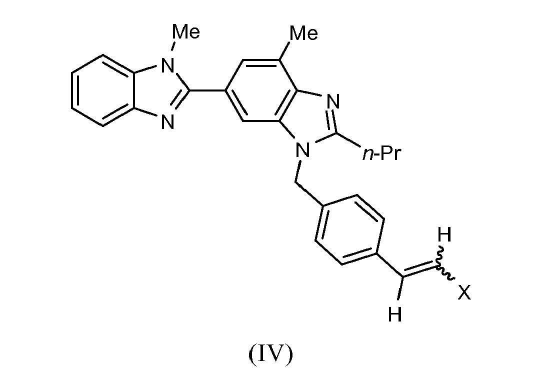 wherein X is a halogen,
with a base B-M,
wherein:
B is a C₁-C₆ alkyl, aryl or R₁O-, where R₁ is C₁-C₆ alkyl, aryl or arylalkyl; M is Li, Na, K or MgX;
in a solvent, to obtain an acetylide of formula (V) 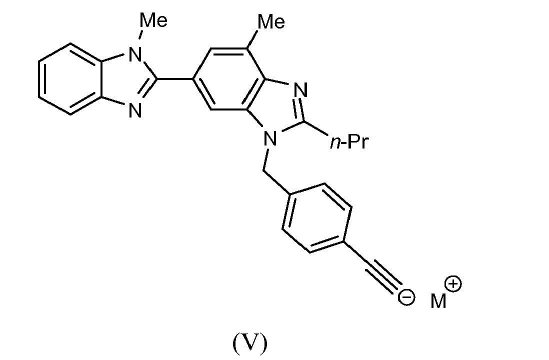
wherein M is as defined above,
followed by reaction with:
a chloroformate of formula (VI) 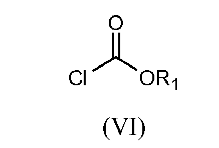
wherein R₁ is as defined above,
to obtain esters of a compound of formula (II), wherein A is COOR₁; and optionally hydrolysis of the esters of a compound of formula (II),
wherein A is COOR₁, and subsequently acidification to obtain the free acid of formula (II), wherein A is COOH.

12. The process according to claim 1, wherein said compound of formula (II) is prepared by reaction of a compound of formula (IV), as defined in claim 11, with a base B-M,
wherein B and M are as defined in claim 11; in a solvent, to obtain an acetylide of formula (V), as defined in claim 11, followed by reaction with CO₂, to obtain salts of compound (II), and optionally acidification to obtain the free acid of formula (II), wherein A is COOH.

13. The process according to claims 11 or 12, wherein the base B-M is an alkyl or aryl metal, a metal alkoxyde, or a Grignard reagent.

14. The process according to claim 13, wherein the base B-M is hexyllithium, sodium or potassium *tert*-butoxide, or isopropylmagnesium chloride.

15. The process according to claims 11 or 12, wherein the solvent is tetrahydrofuran.

16. The process according to claims 11 or 12, wherein said compound of formula
(IV) is prepared by reaction of a compound a formula (VII) 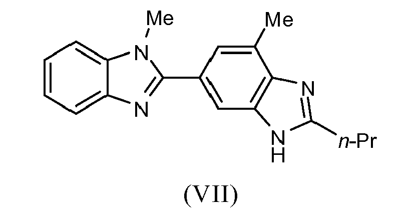 with a compound of formula (VIII), 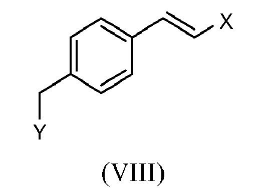 wherein X is a halogen and Y is a leaving group, in the presence of a base and in a solvent.

17. The process according to claim 16, wherein the leaving group is a halogen atom, preferably bromine, or a hydroxyl group activated by esterification with an alkyl or arylsulfonyl group.

18. The process according to claim 16, wherein the base is an inorganic base, preferably potassium carbonate.

19. The process according to claim 16, wherein the solvent is dimethylacetamide.

20. A compound of formula (II), 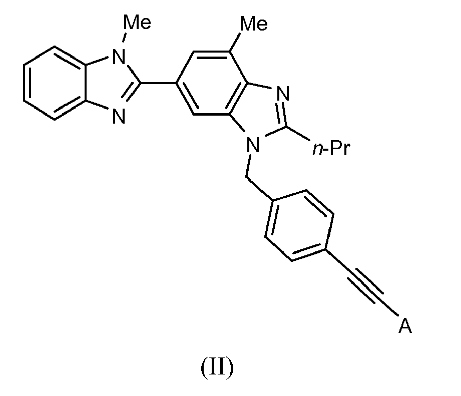 wherein:
A is COOH or COOR₁, where R₁ is C₁-C₆ alkyl, aryl or arylalkyl; as well as its hydrates, solvates, and polymorphs, and the pharmaceutically acceptable salts thereof.

21. A compound according to claim 20, selected from:
3-(4-((1,7'-dimethyl-2'-propyl-1H,3'H-2,5'-bibenzo[d]imidazol-3'-yl)methyl)phenyl) propiolic acid;
sodium 3-(4-((1,7'-dimethyl-2'-propyl-1H,3'H-2,5'-bibenzo[d]imidazol-3'-yl)methyl)phenyl) propiolate;
potassium 3-(4-((1,7'-dimethyl-2'-propyl-1H,3'H-2,5'-bibenzo[d]imidazol-3'-yl)methyl)phenyl) propiolate;
magnesium 3-(4-((1,7'-dimethyl-2'-propyl-1H,3'H-2,5'-bibenzo[d]imidazol-3'-yl)methyl)phenyl) propiolate;
methyl 3-(4-((1,7'-dimethyl-2'-propyl-1H,3'H-2,5'-bibenzo[d]imidazol-3'-yl)methyl)phenyl) propiolate;
ethyl 3-(4-((1,7'-dimethyl-2'-propyl-1H,3'H-2,5'-bibenzo[d]imidazol-3'-yl)methyl)phenyl) propiolate;
isopropyl 3-(4-((1,7'-dimethyl-2'-propyl-1H,3'H-2,5'-bibenzo[d]imidazol-3'-yl)methyl)phenyl) propiolate;
butyl 3-(4-((1,7'-dimethyl-2'-propyl-1H,3'H-2,5'-bibenzo[d]imidazol-3'-yl)methyl)phenyl) propiolate;
phenyl 3-(4-((1,7'-dimethyl-2'-propyl-1H,3'H-2,5'-bibenzo[d]imidazol-3'-yl)methyl)phenyl) propiolate;
as well as its hydrates, solvates, and polymorphs, and the pharmaceutically acceptable salts thereof.

22. A compound of formula (IV) 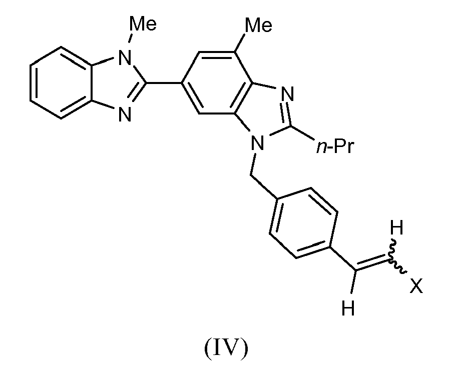 wherein X is a halogen,
as well as its hydrates, solvates, and polymorphs.

23. Use of compounds of formula (II), (IV), as claimed in any one of claims 20-22, as intermediates in the preparation of Telmisartan esters or Telmisartan.
